# FASCICULE DE BREVET EUROPEEN

(11) **EP 0 613 680 B1**
(45) Date de publication et mention de la délivrance du brevet: **29.10.1997**
(21) Numéro de dépôt: 94430001.1
(22) Date de dépôt: 24.02.1994
(51) Int. Cl.: A61K 7/42, A61K 7/48, C07C 49/753, C07C 45/48

(54) **Utilisation de dérivés de 6,6-diméthyl 2 acycyclohex-4-en 1,3-diones dans des compositions de protection solaire**
Verwendung von Derivaten von 6,6 Dimethyl 2-acylcyclohex 4 en 1,3-dionen in Sonnenschutzmitteln
Use of derivatives of 6,6 dimethyl 2-acylcyclohex 4 en 1,3 dione in sunscreening compositions

(30) Priorité: 03.03.1993 FR 9302734; 03.03.1993 FR 9302735
(43) Date de publication de la demande: 07.09.1994
(73) Titulaire: ROBERTET S.A., 06333 Grasse Cédex (FR)
(72) Inventeur: Joulain, Daniel, F-06130 Grasse (FR); Racine, Philippe, F-06520 Magagnosc (FR)
(74) Mandataire: Rinuy, Santarelli

(56) Documents cités:
- FR-A- 2 663 848
- PATENT ABSTRACTS OF JAPAN vol. 16, no. 528 (C-1001) & JP-A-04 198 131 (TAKEDA CHEM IND) 17 Juillet 1992
- S.T.N., Serveur de bases de données, KARLSRUHE, DE, Fichier Chemical Abstracts, vol 75, n 115854 * résumé *
- S.T.N., Serveur de bases de données, KRLSRUHE,DE, Fichier Chemical Abstracts, vol 70, n 14318, * résumé *
- ARKIV FOER KEMI, vol 20, n 1, 1962, STOCKHOLM, pages 1-23, STURE FORSEN, " Molecular orbital calculations of some enolised di- and triketones I. Energies and equilibrium properties. "
- NATURE, vol 241, 1973, LONDON, GB, pages 61-62, LOWRY, " A new Constituent of Biogenetic Pharmaceutical and Historical Interest from Melaleuca Cajeputi Oil. "
- Heilpflanzen-Lexikon für Ärzte und Apotheker, Gustav Fischer Verlag, Stuttgart (DE), 1981, pages 139-140

## Description

La présente invention concerne l'utilisation de 6,6-diméthyl-2-acylcyclohex-4-en-1,3-diones dans le domaine cosmétique de la protection solaire, compositions les renfermant, un nouveau dérivé de la 6,6-diméthyl-2-acylcyclohex-4-en-1,3-dione et son procédé de préparation.

Les produits de protection solaire tels que les crèmes, laits, lotions solaires ou encore les bâtonnets d'application labiale, qu'ils soient destinés à une protection totale ou partielle contre le rayonnement solaire contiennent tous un ou plusieurs filtres solaires.

Ces filtres solaires protecteurs sont pour la plupart d'origine synthétique et utilisés pour absorber sélectivement une frange du spectre des radiations ultraviolettes solaires bien définie. Il s'agit ainsi soit de filtres absorbant les radiations ultraviolettes de type B, soit de filtres absorbant des radiations ultraviolettes de type A courts ou de type A longs.

En effet, jusqu'à une période récente, on a considéré que les radiations ultraviolettes B couvrant la frange de spectre de 290 à 320 nm est la cause majeure des brûlures solaires bien que stimulant la réponse au bronzage, alors que les radiations ultraviolettes couvrant la frange de 320 à 400 nm sont responsables des brûlures de la peau et réactions d'irritation conduisant à la formation de mélanine avec développement du bronzage.

Pour cette raison, la grande majorité des filtres solaires en usage actuellement en cosmétologie n'absorbent que les radiations ultraviolettes de type B.

On peut par exemple citer parmi ce type de filtre de radiations ultraviolettes B le 4-méthoxycinnamate de 2-éthylehexyle qui est couramment utilisé en cosmétologie.

Néanmoins, depuis que le rôle des radiations ultraviolettes A dans l'apparition des cancers cutanés a été reconnu, il s'est avéré nécessaire d'utiliser en combinaison avec des filtres solaires spécifiques des radiations ultraviolettes B, des filtres spécifiques des radiations ultraviolettes A.

Au titre de filtre des radiations ultraviolettes A sont généralement utilisés des dérivés du dibenzoylméthane qui absorbent les radiations ultraviolettes A vers 350 nm.

A ce jour, il n'a pas encore été proposé de filtre solaire qui à lui seul puisse absorber non seulement les radiations ultraviolettes B mais aussi une frange très importante du spectre des radiations ultraviolettes A.

Les filtres solaires utilisés actuellement sont tous des filtres sélectifs soit des radiations ultraviolettes A et très faiblement des radiations ultraviolettes B, soit des radiations ultraviolettes B et très faiblement des radiations ultraviolettes A.

La demanderesse a découvert avec étonnement que certains dérivés de la famille des 6,6-diméthyl-2-acylcyclohex-4-en-1,3-diones présentaient non seulement la faculté d'absorber les radiations ultraviolettes B mais aussi une partie très importante des radiations ultraviolettes A.

Il s'agit des membres de la famille répondant à la formule (I) 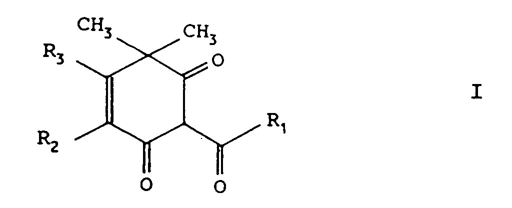 dans laquelle R₁ est un radical alkyle en C₁ à C₆, R₂ est l'atome d'hydrogène ou un radical alkyle, R₃ est un atome d'hydrogène, un radical hydroxy ou un radical alcoxy.

A titre d'exemples parmi cette famille on peut citer par exemple la 4,6,6-triméthyl-5-méthoxy-2-acétylcyclohex-4-en-1,3-dione présentant notamment un maximum d'absorption à 320 nm, la 6,6-diméthyl-5-méthoxy-2-isobutyrylcyclohex-4-en-1,3-dione présentant également un des maxima d'absorption à 322 nm mais aussi la 4,6,6-triméthyl-5-méthoxy-2-isobutyrylcyclohex-4-en-1,3-dione dont un des maxima d'absorption est situé à 324 nm.

C'est pourquoi la présente invention a pour objet l'utilisation d'une 6,6 diméthyl-2-acylcyclohex-4-en-1,3-dione de formule (I) 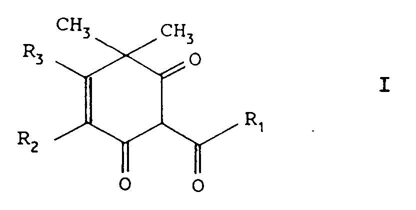 dans laquelle R₁ est un radical alkyle en C₁-C₆, R₂ est l'atome d'hydrogène ou un radical alkyle en C₁-C₆, R₃ est un atome d'hydrogène, un radical hydroxy ou un radical alcoxy en C₁-C₆, à titre de principe actif en cosmétique et notamment de filtre solaire.

Dans la formule (I) et dans ce qui suit, par radical alkyle en C₁-C₆, l'on entend un radical tant linéaire que ramifié par exemple le radical méthyl, éthyl, n-propyl, méthyléthyle, n-butyle ou n-hexyle, 1,1 diméthyl éthyle; par radical alcoxy en C₁-C₆, l'on entend un radical linéaire ou ramifié, par exemple méthoxy, éthoxy ou n-propoxy.

Parmi cette famille de dérivés certains sont présents à l'état naturel. On peut citer notamment :
- la 4,6,6-triméthyl-5-méthoxy-2-acétylcyclohex-4-en-1,3-dione (où donc R₁ et R₂ sont des radicaux méthyle et R₃ un radical méthoxy dans la formule (I)), extraite des feuilles de Melaleuca cajeputi, arbre de la famille des myrtacées qui croît notamment en Malaisie péninsulaire, ainsi que dans l'île de Sumatra en Indonésie ;
- la 4,6,6-triméthyl-5-hydroxy-2-n-butyrylcyclohex-4-en-1,3-dione, aussi intitulée fraginol, (où donc R₁ est un radical propyl, R₂ est un radical méthyl et R₃ est un radical hydroxy dans la formule (I)), extraite de Dryopteris fragrans (L.M MOLODOZHNIKOVA et Coll, Khim-Farm.Zh., 1971, 5, 32) ;
- la 4,6,6-triméthyl-5-méthoxy-2-isobutyrylcyclohex-4-en-1,3-dione, aussi intitulée tasmanone, (où donc R₁ est un radical isopropyl, R₂ un radical méthyl et R₃ un radical méthoxy) présente dans l'Eucalyptus Tasmanica (R.O HELLYER et Coll, Australian Journal of Chemistry, 1956, 9, 238) ;
- la 6,6-diméthyl-5-méthoxy-2-isobutyrylcyclohex-4-en-1,3-dione, aussi intitulée agglomérone (où donc R₁ est un radical isopropyl, R₂ est l'hydrogène et R₃ est un radical méthoxy dans la formule (I)), extraite des huiles essentielles d'Eucalyptus agglomerata et d'Eucalyptus Mc Kieana (R.O HELLYER et Coll, Australian Journal of Chemistry, 1964, 17, 1418) ;
- la 6,6-diméthyl-2-isobutyrylcyclohex-4-en-1,3-dione, intitulée xanthostémone, analogue déméthylé de l'agglomérone, extraite de Xanthostemon appositifolius (A.J. BIRCH et Coll, Australian Journal of Chemistry, 1956, 9, 238).

Comme dit précédemment, les filtres solaires utilisés à ce jour sont essentiellement d'origine synthétique. Leur remplacement par des substances naturelles se révèle particulièrement avantageux dans l'utilisation à titre de filtre solaire car le filtre solaire étant par définition d'application corporelle, les utilisateurs sont à ce titre de plus en plus demandeurs de substances naturelles.

A titre de filtre solaire la famille de dérivés répondant à la formule (I) dans laquelle R₁ est un radical alkyle en C₁ à C₆, R₂ est l'atome d'hydrogène ou un radical alkyle, R₃ est un atome d'hydrogène, un radical hydroxy ou un radical alcoxy, peut être incorporée à des compositions de protection solaire telles que crèmes solaires écran total ou haute protection.

Il peut également s'agir des laits, lotions, bâtonnets d'application labiale ou gels correspondants.

C'est pourquoi la présente invention a aussi pour objet une crème, un gel, un lait, une lotion ou un bâtonnet d'application labiale renfermant au moins un filtre solaire, caractérisé en ce qu'il renferme à titre de filtre solaire au moins un dérivé de la 6,6-diméthyl-2-acylcyclohex-4-en-1,3-dione de formule (I), dans laquelle R₁ est un radical alkyle en C₁-C₆, R₂ est l'atome d'hydrogène ou un radical alkyle en C₁-C₆ , R₃ est un atome d'hydrogène, un radical hydroxy ou un radical alcoxy en C₁-C₆.

Avantageusement, dans la formule (I) R₃ est un radical alcoxy et de préférence un radical méthoxy pour l'utilisation à titre de filtre solaire ou dans les compositions renfermant le filtre solaire.

Avantageusement également l'utilisation ou les compositions sont caractérisées en ce que R₁ est un radical méthyle ou isopropyle, R₂ est un atome d'hydrogène ou un radical méthyle, R₃ est un radical méthoxy.

De préférence on choisira pour l'utilisation à titre de filtre solaire ou dans une composition correspondante, un dérivé de formule I choisi parmi :
- la 4,6,6-triméthyl-5-méthoxy-2-acétylcyclohex-4-en-1,3-dione,
- la 4,6,6-triméthyl-5-hydroxy-2-n-butyrylcyclohex-4-en-1,3-dione,
- la 4,6,6-triméthyl-5-méthoxy-2-isobutyrylcyclohex-4-en-1,3-dione,
- la 6,6-diméthyl-5-méthoxy-2-isobutyrylcyclohex-4-en-1,3-dione, et
- la 6,6-diméthyl-2-isobutyrylcyclohex-4-en-1,3-dione.

Par ailleurs, il est à noter que les dérivés de la présente invention soumis à une tautomérie céto-énolique peuvent être mis en oeuvre sous l'une quelconque de leurs formes tautomères.

Sture Forsén a imaginé un certain nombre de formules de dérivés di- et tricétoniques dont il a dessiné la formule et a également, de manière théorique, calculé un certain nombre de valeurs d'énergie totale π électronique et de délocalisation. Parmi les formules qu'il a imaginées,publiées dans Arkiv for kemi Vol.20 n°1, 1962, figure la 4,6,6-triméthyl-5-méthoxy-2-acétylcyclohex-4-en-1,3-dione ci-dessus. Aucune constante n'a été déterminée en pratique pour ce produit, ni aucun procédé de préparation n'est donné pour ce produit.

La 4,6,6-triméthyl-5-méthoxy-2-acétylcyclohex-4-en-1,3-dione doit être considérée comme un nouveau produit naturel. C'est pourquoi la présente demande a aussi pour objet la 4,6,6-triméthyl-5-méthoxy-2-acétylcyclohex-4-en-1,3-dione répondant à la formule développée II

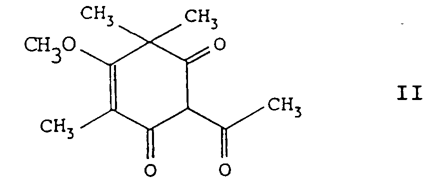

La 4,6,6-triméthyl-5-méthoxy-2-acétylcyclohex-4-en-1,3-dione est sujette à une tautomérie céto-énolique ; à l'état naturel, elle existerait sous ses deux formes tautomères énoliques (IIa) et (IIb) selon l'équilibre prototropique suivant dans la proportion de 65,3 % de (IIa) et 34,7% de (IIb):

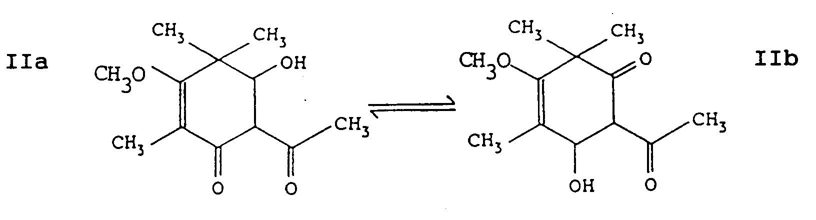

La formule (II) et sa dénomination représenteront dans la suite du texte une simplification d'écriture désignant la 4,6,6-triméthyl-5-méthoxy-2-acétylcyclohex-4-en-1,3-dione sous n'importe laquelle de ses formes tautomères notamment les formes (IIa) et (IIb).

La présente invention a donc aussi pour objet la 4,6,6-triméthyl-5-méthoxy-2-acétylcyclohex-4-en-1,3-dione de formule (II), notamment sous ses formes tautomères de formules (IIa) et (IIb).

Le dérivé précité préféré se trouve notamment sous forme isolée, particulièrement sous une forme substantiellement pure.

La présente demande a également pour objet un procédé d'obtention de la 4,6,6-triméthyl-5-méthoxy-2-acétylcyclohex-4-en-1,3-dione caractérisé en ce qu'il consiste à :
(a) ajuster le pH d'une solution d'énolates alcalins de formule (II) de façon à libérer les énols correspondants;
(b) isoler par extraction au moyen d'un solvant, de préférence le dichlorométhane, lesdits énols, et
(c) éventuellement purifier lesdits énols par distillation fractionnée sous pression réduite.

La solution d'énolates alcalins ci-dessus peut être préparée comme suit :
(d) on soumet du feuillage de Melaleuca cajeputi, soit frais ou sec à un entraînement à la vapeur, soit partiellement desséché à une extraction au moyen d'un solvant ;
(e) on soumet le produit obtenu dans l'étape (d), en solution dans un solvant, à une extraction au moyen d'un carbonate alcalin.

Avantageusement le solvant des étapes (d) et (e) du procédé ci-dessus est le tertiobutylméthyléther, l'acétate d'éthyle ou, de préférence, l'hexane.

La température d'extraction de l'étape (d) sera avantageusement choisie entre 40°C et 60°C.

Le carbonate alcalin de l'étape (e) est de préférence le carbonate de sodium.

De préférence également le pH de l'étape (a) est ajusté entre 6 et 7, avantageusement à l'aide d'un acide, de préférence minéral tel l'acide chlorhydrique ou l'acide phosphorique.

La 4,6,6-triméthyl-5-méthoxy-2-acétylcyclohex-4-en-1,3-dione ayant également une très bonne activité antibactérienne et antifongique, la présente invention a par ailleurs aussi pour objets respectifs l'application de la 4,6,6-triméthyl-5-méthoxy-2-acétylcyclohex-4-en-1,3-dione à titre d'agent antibactérien et d'agent antifongique.

A titre de principe actif médicamenteux, notamment d'agent antibactérien ou antifongique, la 4,6,6-triméthyl-5-méthoxy-2-acétylcyclohex-4-en-1,3-dione peut aussi être incorporée dans des compositions cosmétiques telles que les déodorants corporels ou les autres produits d'hygiène corporelle, ou dans des compositions pharmaceutiques.

La présente invention a donc encore pour objet les compositions cosmétiques ne renfermant pas de filtre solaire, ainsi que les compositions pharmaceutiques caractérisées en ce qu'elles renferment à titre de principe actif la 4,6,6-triméthyl-5-méthoxy-2-acétylcyclohex-4-en-1,3-dione.

La 4,6,6-triméthyl-5-méthoxy-2-acétylcyclohex-4-en-1,3-dione trouve notamment son emploi dans le traitement tant préventif que curatif des affections liées aux germes sur lesquels elle est active, telles que les candidoses et d'autres affections fongiques.

Ces compositions pharmaceutiques ou cosmétiques peuvent être, par exemple, pâteuses ou liquides et se présenter sous les formes couramment utilisées en application topique, comme par exemple les crèmes ou les gels ; elles sont préparées selon les méthodes usuelles. Le ou les principes actifs peuvent y être incorporés à des excipients habituellement employés dans ces compositions, tels que le beurre de cacao, les véhicules aqueux ou non, les corps gras d'origine animale ou végétale, les dérivés paraffiniques, les glycols, les divers agents mouillants, dispersants ou émulsifiants, les conservateurs.

Les exemples qui suivent illustrent la présente invention :

### EXEMPLE 1: Crème solaire

On a préparé une crème solaire haute protection renfermant à titre de principe actif la 4,6,6-triméthyl-5-méthoxy-2-acétylcyclohex-4-en-1,3-dione et comme excipient une émulsion huile dans eau contenant :
- des esters gras,
- de l'huile de vaseline,
- un polymère carboxyvinylique,
- de l'imidazoldinylurée, et
- un parfum testé hypoallergénique.

Le principe actif représente 1 à 3% de l'ensemble de la composition.

A titre de comparaison, on a consigné dans le tableau suivant, les coefficients d'absorbance du principe actif de la composition susmentionnée et du 4-méthoxycinnamate de 2-éthylhexyle qui est un filtre solaire de radiations ultraviolettes B couramment utilisé en cosmétologie.

Ces coefficients sont d'une part ceux correspondant à chacun des maxima d'absorption respectif et d'autre part ceux correspondant à la longueur d'onde de 350 nm pour chacun des filtres solaires.

Ils sont obtenus à partir d'un spectre ultraviolet, le solvant utilisé étant le méthanol.

| Substance | ε max | ε 350 nm |
|---|---|---|
| 4,6,6-triméthyl-5-méthoxy-2-acétylcyclohex-4-en-1,3-dione (λ max = 320 nm) | 480 | 370 |
| 4-méthoxycinnamate de 2-éthylhexyle (λ max = 310 nm) | 607 | 42,6 |

Il apparaît clairement qu'à 350 nm, longueur d'onde à laquelle les filtres sélectifs des radiations ultraviolettes A du type dibenzoylméthane sont actifs, le coefficient d'absorbance de la 4,6,6-triméthyl-5-méthoxy-2-acétylcyclohex-4-en-1,3-dione est 8,7 fois plus élevé dans les mêmes conditions que celui du filtre des radiations ultraviolettes B classique.

Par ailleurs, la performance de la 4,6,6-triméthyl-5-méthoxy-2-acétylcyclohex-4-en-1,3-dione comme filtre des radiations ultraviolettes B est très attrayante si on la compare à celle du filtre sélectif de radiations ultraviolettes B classique.

### EXEMPLE 2 : Préparation de la 4,6,6-triméthyl-5-méthoxy-2-acétylcyclohex-4-en-1,3-dione à partir de l'huile essentielle extraite de feuilles de Melaleuca cajeputi.

**Stade A :** Dans un alambic en acier inoxydable, à double fond, d'une capacité de 300 litres, on charge 45 kg de feuilles de Melaleuca cajeputi. On soumet à un entraînement à la vapeur pendant 6 heures. On recueille 327 g d'huile essentielle de couleur jaune foncé (rendement 0,72 %).
**Stade B :** On dissout 462 g d'huile essentielle telle qu'obtenue ci-dessus dans 1 litre d'hexane. On soumet cette solution à 8 extractions successives avec 500 ml de solution aqueuse de carbonate de sodium à 10%. Les extraits carbonatés sont réunis et utilisés tels quels pour le stade suivant.
**Stade C :** Les extraits carbonatés obtenus ci-dessus sont à leur tour épuisés à deux reprises avec 250 ml d'hexane, de façon à éliminer toute matière organique neutre. On amène le pH de la phase aqueuse à 6,5 environ, par addition de la quantité suffisante d'acide chlorhydrique à 50 %.

On extrait alors la phase aqueuse à trois reprises avec 500 ml de dichlorométhane. Les phases organiques réunies sont débarrassées du solvant par évaporation, livrant finalement 141 g d'un produit brut de couleur jaune. On distille ce produit dans une colonne de Vigreux de 15 cm de hauteur, et recueille 130 g de distillat jaune clair, sous la forme d'un liquide mobile, bouillant à 133 °C sous la pression de 0,67 KPa. La pureté est estimée à 99,9 % minimum par chromatographie en phase gazeuse. La densité à 20°C par rapport à l'eau prise à 20°c est de 1,152 et son indice de réfraction à 20°C est de 1,546.

Le spectre infrarouge de la 4,6,6-triméthyl-5-méthoxy-2-acétylcyclohex-4-en-1,3-dione présente des bandes intenses caractéristiques à 1663 cm⁻¹, 1640 cm⁻¹, 1540 cm⁻¹, 1465 cm⁻¹ et 1135 cm⁻¹.

Son spectre ultraviolet dans le méthanol présente quant à lui deux maximum d'absorption à 242 nm et 320 nm.

Les coefficients d'absorbance respectifs sont de 10940 et 6030.

Le spectre RMN du proton 1H fait l'objet du tableau 1 ci-dessous. Il a été obtenu dans le chloroforme deutéré à 400MHz. Les déplacements δ sont exprimées en partie par million par rapport au TMS.

On observera que tous les pics sortent sous forme de singulet et que le spectre du proton permet de distinguer d'après leurs déplacements, les deux formes tautomères énoliques (Ia) et (Ib), au sein du mélange obtenu par le procédé précédemment décrit.

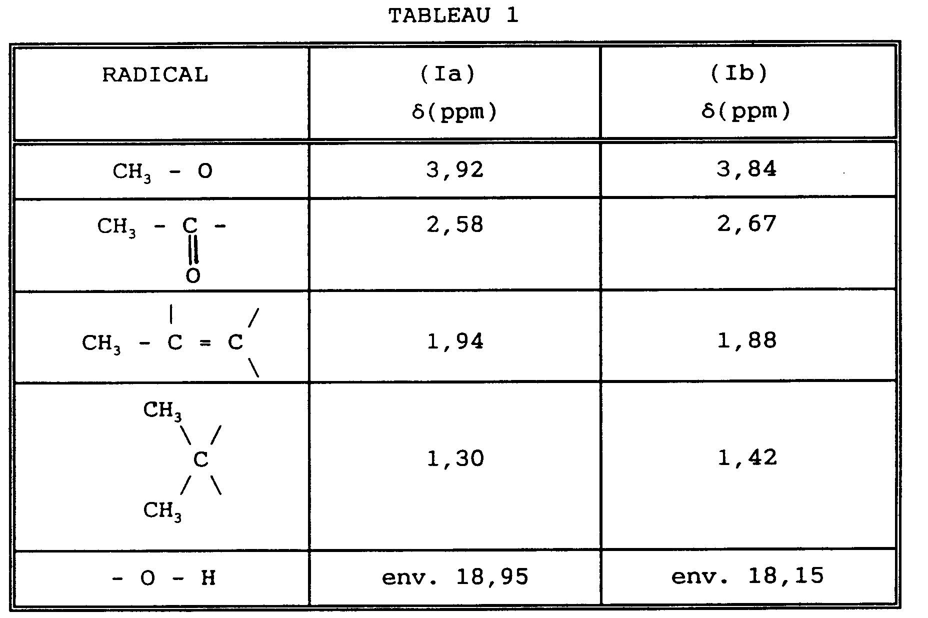

Il en est de même pour cette distinction dans le cas du RMN du carbone 13 réalisé à 161 MHz dans le chloroforme deutéré et consigné dans le tableau 2 suivant.

La numérotation des carbones a été effectuée en référence à la formule suivante :

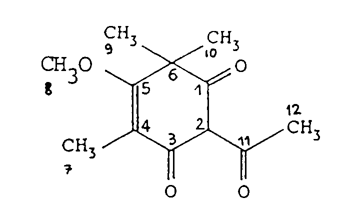

**TABLEAU 2**

| Atome de carbone | (Ia) δ (ppm) | (Ib) δ (ppm) |
|---|---|---|
| C - 1 | 197,7 (a) | 197,6 (a) |
| C - 2 | 107,6 | 109,9 |
| C - 3 | 190,6 | 185,9 |
| C - 4 | 112,4 | 118,4 |
| C - 5 | 177,4 | 170,9 |
| C - 6 | 50,6 | 45,4 |
| C - 7 | 10,3 (b) | 9,9 (b) |
| C - 8 | 62,6 | 62,3 |
| C - 9 | 24,7 (c) | 24,7 (c) |
| C - 10 | 24,6 (c) | 24,6 (c) |
| C - 11 | 201,6 | 204,4 |
| C - 12 | 28,4 | 29,7 |

On notera ici que les valeurs référencées (a), (b) et (c) étant identiques ou très proches, il n'est pas possible de les attribuer avec certitude à des atomes de carbones définis : elles sont interchangeables.

Le tableau 3 illustre le spectre de masse du mélange des formes tautomères (Ia) et (Ib) de la 4,6,6-triméthyl-5-méthoxy-2-acétylcyclohex-4-en-1,3-dione.

Les rapports de la masse sur la charge m/z des différents pics y sont consignés ainsi que l'intensité relative de chacun des pics correspondants.

**TABLEAU 3**

| m / z | % |
|---|---|
| 43 | 100 |
| 81 | 61 |
| 224 | 39 |
| 41 | 34 |
| 209 | 31 |
| 139 | 25 |
| 192 | 25 |
| 125 | 23 |
| 67 | 20 |
| 69 | 19 |

### EXEMPLE 3 : Une autre application particulièrement avantageuse de la 4,6,6-triméthyl-5-méthoxy-2-acétylcyclohex-4-en-1,3-dione est celle à titre d'agent antibactérien ou antifongique.

En effet, comme illustré dans le tableau 5, l'activité antibactérienne de la 4,6,6-triméthyl-5-méthoxy-2-acétylcyclohex-4-en-1,3-dione a été testée in vitro sur divers microorganismes aux concentrations de 0,1 % et 0,2 % dans un milieu de culture qui est ici de la gélose.

L'activité a été estimée sur une échelle allant de 0 (aucune activité) à 5 (inhibition totale de la croissance des germes).

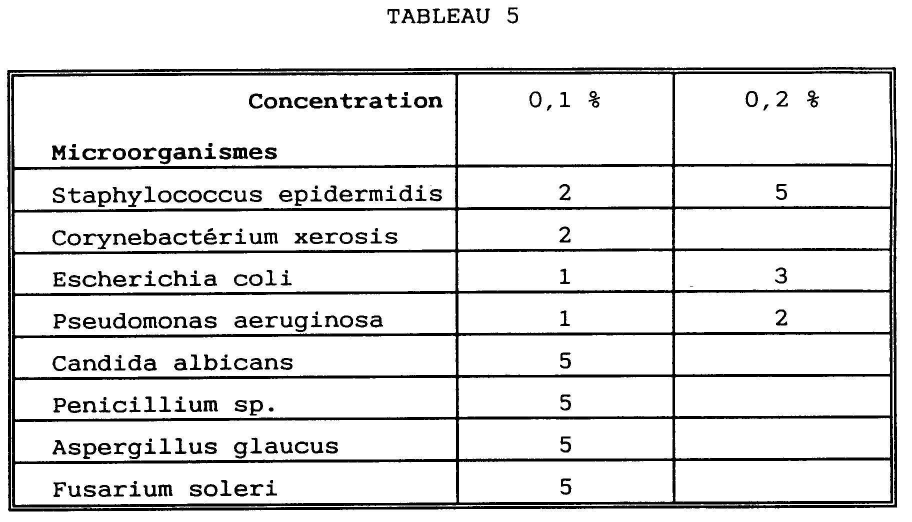

Ces résultats confirment que la 4,6,6-triméthyl-5-méthoxy-2-acétylcyclohex-4-en-1,3-dione possède une bonne activité antimicrobienne, particulièrement antibactérienne et antifongique et peut donc être utilisée avantageusement à ce titre.

En particulier, du fait de son activité sur les bactéries de la flore axillaire, combinée à sa faible volatilité (sa pression de vapeur à 25 °C étant voisine de 1,5 micromètres de mercure), la 4,6,6-triméthyl-5-méthoxy-2-acétylcyclohex-4-en-1,3-dione a un bon pouvoir de déodorant corporel.

Elle peut ainsi être appliquée à une composition cosmétique telle qu'un déodorant corporel dans les proportions suivantes en poids :

### EXEMPLE 4 : Composition cosmétique

On a préparé un déodorant corporel selon la formule suivante :

| | |
|---|---|
| - éthanol à 96° | 72 % |
| - 4,6,6-triméthyl-5-méthoxy-2-acétylcyclohex-4-en-1,3-dione | 0,2 % |
| - eau déminéralisée | 27,8 % |

Cette préparation assure une bonne activité déodorante corporelle d'une durée moyenne de 12 heures pour la majorité des sujets.

Un tel déodorant rejoint les buts fixés pour la présente invention, en ce sens qu'il est composé de substances qui sont toutes naturelles.

### EXEMPLE 5 : Variante de l'exemple 2.

On soumet le feuillage partiellement desséché de Melaleuca cajeputi à une extraction par l'hexane vers 50°C, à l'aide d'un extracteur statique tel que ceux qui sont habituellement utilisés dans l'industrie des matières premières pour la parfumerie, puis on soumet le produit obtenu, en solution dans l'hexane, à une extraction au moyen de carbonate de sodium. On continue alors le procédé comme indiqué au stade (C) ci-dessus pour obtenir le produit attendu.

### EXEMPLE 6 : Composition cosmétique

On a préparé une crème ayant la composition pondérale suivante :

| Part A | |
|---|---|
| Huile de germe de blé | 0,5 |
| Glycéryl stéarate ceteareth 20 | 7,0 |
| PEG 30 glycéryl stéarate | 5,0 |
| Alcool Cétylique | 1,0 |
| Propyl Paraben | 0,1 |
| BHT | 0,05 |
| Huile de paraffine | 6,0 |
| Substance (I) | 3,0 |

| Part B | |
|---|---|
| Eau déminéralisée | 60,0 |
| Méthyl Paraben | 0,15 |
| Carbopol 940 | 0,2 |
| Glycérine | 5,0 |

| Part C | |
|---|---|
| Eau déminéralisée | 11,84 |
| Triéthanolamine | 0,16 |

### MODE OPERATOIRE

Dans un fondoir en acier inoxydable, on mélange les ingrédients de la phase grasse (A) en portant la température à 80°C.

Dans un autre fondoir on verse le propyl Paraben, le Carbopol en pluie dans l'eau portée à 80°C (sous forte agitation), puis la glycérine.

On ajoute lentement la phase grasse (60°C) dans la phase aqueuse (B) (60°C), sous agitation (en évitant l'introduction d'air).

Quand l'émulsion commence à épaissir, on verse lentement la phase (C), puis si désiré un parfum à la dose de 0,2-0,5 % et on homogénéise.

## Revendications

1. Utilisation d'un composé dérivé de la 6,6 diméthyl-2-acylcyclohex-4-en-1,3-dione de formule (I) 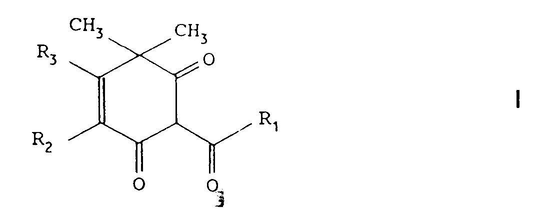 dans laquelle R₁ est un radical alkyle en C₁-C₆, R₂ est l'atome d'hydrogène ou un radical alkyle en C₁-C₆, R₃ est un atome d'hydrogène, un radical hydroxy ou un radical alcoxy en C₁-C₆, à titre de filtre solaire.

2. Utilisation selon la revendication 1, caractérisée en ce que R₃ est un radical alcoxy en C₁-C₆.

3. Utilisation selon la revendication 1 ou 2, caractérisée en ce que R₃ est un radical méthoxy.

4. Utilisation selon la revendication 1, 2 ou 3 caractérisée en ce que R₁ est un radical méthyle ou isopropyle, R₂ est un atome d'hydrogène ou un radical méthyle, R₃ est un radical méthoxy.

5. Utilisation selon la revendication 1, caractérisée en ce que le composé est choisi parmi :
- la 4,6,6-triméthyl-5-méthoxy-2-acétylcyclohex-4-en-1,3-dione,
- la 4,6,6-triméthyl-5-hydroxy-2-n-butyrylcyclohex-4-en-1,3-dione,
- la 4,6,6-triméthyl-5-méthoxy-2-isobutyrylcyclohex-4-en-1,3-dione,
- la 6,6-diméthyl-5-méthoxy-2-isobutyrylcyclohex-4-en-1,3-dione, et
- la 6,6-diméthyl-2-isobutyrylcyclohex-4-en-1,3-dione.

6. Une crème, un gel, un lait, une lotion ou un bâtonnet d'application labiale renfermant au moins un filtre solaire caractérisé en ce qu'il renferme à titre de principe actif au moins un dérivé de la 6,6-diméthyl-2-acylcyclohex-4-en-1,3-dione de formule (I) 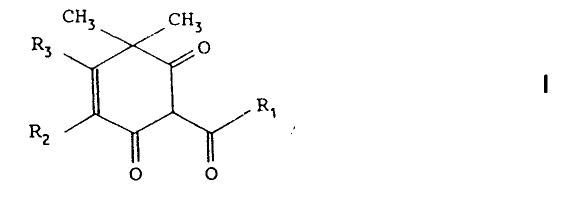 dans laquelle R₁ est un radical alkyle en C₁-C₆, R₂ est l'atome d'hydrogène ou un radical alkyle en C₁-C₆, R₃ est un atome d'hydrogène, un radical hydroxy ou un radical alcoxy en C₁-C₆.

7. Une crème, un gel , un lait, une lotion ou un bâtonnet d'application labiale selon la revendication 6, caractérisé en ce que R₃ est un radical alcoxy en C₁-C₆.

8. Une crème, un gel , un lait, une lotion ou un bâtonnet d'application labiale selon la revendication 6 ou 7, caractérisé en ce que R₃ est un radical méthoxy.

9. Une crème, un gel , un lait, une lotion ou un bâtonnet d'application labiale selon la revendication 6, 7 ou 8 caractérisé en ce que R₁ est un radical méthyle ou isopropyle, R₂ est un atome d'hydrogène ou un radical méthyle, R₃ est un radical méthoxy.

10. Une crème, un gel , un lait, une lotion ou un bâtonnet d'application labiale selon la revendication 6, caractérisé en ce que le composé de formule I est choisi parmi :
- la 4,6,6-triméthyl-5-méthoxy-2-acétylcyclohex-4-en-1,3-dione,
- la 4,6,6-triméthyl-5-hydroxy-2-n-butyrylcyclohex-4-en-1,3-dione,
- la 4,6,6-triméthyl-5-méthoxy-2-isobutyrylcyclohex-4-en-1,3-dione,
- la 6,6-diméthyl-5-méthoxy-2-isobutyrylcyclohex-4-en-1,3-dione, et
- la 6,6-diméthyl-2-isobutyrylcyclohex-4-en-1,3-dione.

11. La 4,6,6-triméthyl-5-méthoxy-2-acétylcyclohex-4-en-1,3-dione de formule (II) : 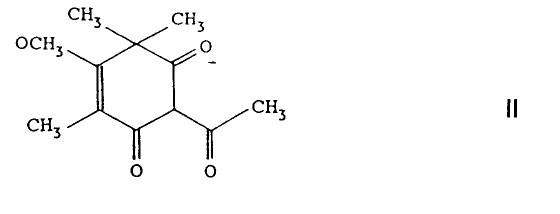 sous l'une quelconque de ses différentes formes tautomères.

12. Procédé de préparation de la 4,6,6-triméthyl-5-méthoxy-2-acétylcyclohex-4-en-1,3-dione caractérisé en ce qu'il consiste à :
(a) ajuster le pH d'une solution d'énolates alcalins de la 4,6,6-triméthyl-5-méthoxy-2-acétylcyclohex-4-en-1,3-dione de façon à libérer les énols correspondants;
(b) isoler par extraction lesdits énols au moyen d'un solvant, et
(c) éventuellement purifier lesdits énols par distillation fractionnée sous pression réduite.

13. Procédé selon la revendication 12, caractérisé en ce que la solution d'énolates alcalins est préparée selon un procédé qui consiste à :
(d) soumettre du feuillage de Melaleuca cajeputi, soit frais ou sec à un entraînement à la vapeur, soit partiellement desséché à une extraction au moyen d'un solvant ;
(e) soumettre le produit obtenu dans l'étape (d), en solution dans un solvant, à une extraction au moyen d'un carbonate alcalin.

## Claims

1. Use of a compound derived from 6,6-dimethyl-2-acylcyclohex-4-en-1,3-dione of the formula (I) 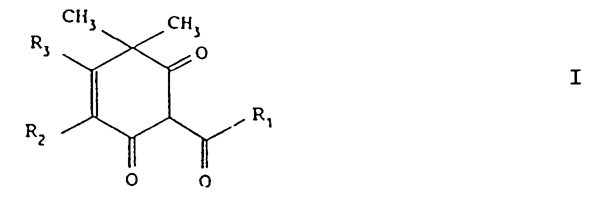 in which R₁ is a C₁-C₆ alkyl radical, R₂ is a hydrogen atom or a C₁-C₆ alkyl radical, R₃ is a hydrogen atom, a hydroxy radical or a C₁-C₆ alkoxy radical as a sunscreen.

2. Use according to claim 1, characterised in that R₃ is a C₁-C₆ alkoxy radical.

3. Use according to claim 1 or 2, characterised in that R₃ is a methoxy radical.

4. Use according to claim 1, 2 or 3, characterised in that R₁ is a methyl or isopropyl radical, R₂ is a hydrogen atom or a methyl radical, R₃ is a methoxy radical.

5. Use according to claim 1, characterised in that the compound is selected from:
- 4,6,6-trimethyl-5-methoxy-2-acetylcyclohex-4-en-1,3-dione,
- 4,6,6-trimethyl-5-hydroxy-2-n-butyrylcyclohex-4-en-1,3-dione,
- 4,6,6-trimethyl-5-methoxy-2-isobutyrylcyclohex-4-en-1,3-dione,
- 6,6-dimethyl-5-methoxy-2-isobutyrylcyclohex-4-en-1,3-dione and
- 6,6-dimethyl-2-isobutyrylcyclohex-4-en-1,3-dione.

6. A cream, a gel, a milk, a lotion or a stick for application to the lips containing at least one sunscreen, characterised in that it contains as active ingredient at least one derivative of 6,6-dimethyl-2-acylcyclohex-4-en-1,3-dione of the formula (I) 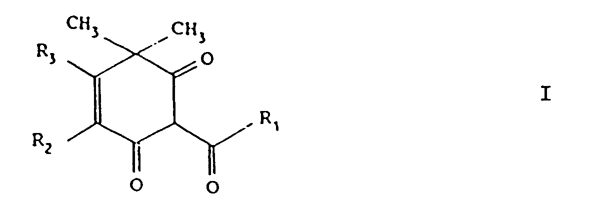 in which R₁ is a C₁-C₆ alkyl radical, R₂ is a hydrogen atom or a C₁-C₆ alkyl radical, R₃ is a hydrogen atom, a hydroxy radical or a C₁-C₆ alkoxy radical.

7. A cream, a gel, a milk, a lotion or a stick for application to the lips according to claim 6, characterised in that R₃ is a C₁-C₆ alkoxy radical.

8. A cream, a gel, a milk, a lotion or a stick for application to the lips according to claim 6 or 7, characterised in that R₃ is a methoxy radical.

9. A cream, a gel, a milk, a lotion or a stick for application to the lips according to claim 6, 7 or 8, characterised in that R₁ is a methyl or isopropyl radical, R₂ is a hydrogen atom or a methyl radical, R₃ is a methoxy radical.

10. A cream, a gel, a milk, a lotion or a stick for application to the lips according to claim 6, characterised in that the compound of formula I is selected from:
- 4,6,6-trimethyl-5-methoxy-2-acetylcyclohex-4-en-1,3-dione,
- 4,6,6-trimethyl-5-hydroxy-2-n-butyrylcyclohex-4-en-1,3-dione,
- 4,6,6-trimethyl-5-methoxy-2-isobutyrylcyclohex-4-en-1,3-dione,
- 6,6-dimethyl-5-methoxy-2-isobutyrylcyclohex-4-en-1,3-dione and
- 6,6-dimethyl-2-isobutyrylcyclohex-4-en-1,3-dione.

11. 4,6,6-trimethyl-5-methoxy-2-acetylcyclohex-4-en-1,3-dione of the formula (II): 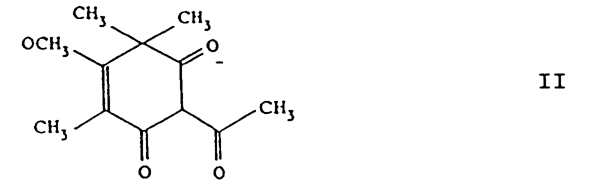 in any one of its various tautomeric forms.

12. Process for the production of 4,6,6-trimethyl-5-methoxy-2-acetylcyclohex-4-en-1,3-dione, characterised in that it consists in:
(a) adjusting the pH of a solution of alkali enolates of 4,6,6-trimethyl-5-methoxy-2-acetylcyclohex-4-en-1,3-dione in such a manner as to release the corresponding enols;
(b) isolating the said enols by extraction with a solvent and
(c) optionally purifying the said enols by fractional distillation under reduced pressure.

13. Process according to claim 12, characterised in that the solution of alkali enolates is prepared according to a process consisting in:
(a) subjecting foliage of *Melaleuca cajeputi*, if fresh or dry, to steam distillation or, if partially dried, to solvent extraction;
(b) extracting the product obtained from stage (d), dissolved in a solvent, with an alkali carbonate.

## Patentansprüche

1. Verwendung einer von 6,6-Dimethyl-2-acylcyclohex-4-en-1, 3-dion der Formel (I) abgeleiteten Verbindung, 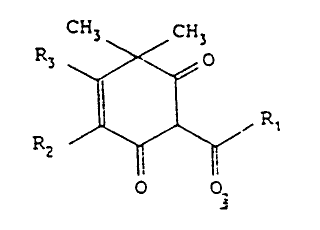 worin R₁ für einen C₁-C₆-Alkylrest, R₂ für ein Wasserstoffatom oder einen C₁-C₆-Alkylrest und R₃ für ein Wasserstoffatom, eine Hydroxygruppe oder einen C₁-C₆-Alkoxyrest steht, als Sonnenfilter.

2. Verwendung nach Anspruch 1, dadurch gekennzeichnet, daß R₃ für einen C₁-C₆-Alkoxyrest steht.

3. Verwendung nach Anspruch 1 oder 2, dadurch gekennzeichnet, daß R₃ für einen Methoxyrest steht.

4. Verwendung nach Anspruch 1, 2 oder 3, dadurch gekennzeichnet, daß R₁ für einen Methyl- oder Isopropylrest, R₂ für ein Wasserstoffatom oder einen Methylrest und R₃ für einen Methoxyrest steht.

5. Verwendung nach Anspruch 1, dadurch gekennzeichnet, daß die Verbindung ausgewählt ist aus:
- 4,6,6-Trimethyl-5-methoxy-2-acetylcyclohex-4-en-1,3-dion,
- 4,6,6-Trimethyl-5-hydroxy-2-n-butyrylcyclohex-4-en-1,3-dion,
- 4,6,6-Trimethyl-5-methoxy-2-isobutyrylcyclohex-4-en-1,3-dion,
- 6,6-Dimethyl-5-methoxy-2-isobutyrylcyclohex-4-en-1,3-dion und
- 6,6-Dimethyl-2-isobutyrylcyclohex-4-en-1,3-dion.

6. Creme, Gel, Milch, Lotion oder Lippenstift, der/die mindestens einen Sonnenfilter enthält, dadurch gekennzeichnet, daß er/sie als Wikstoff mindestens ein Derivat des 6,6-Dimethyl-2-acylcyclohex-4-en-1,3-dions der Formel (I) enthält, 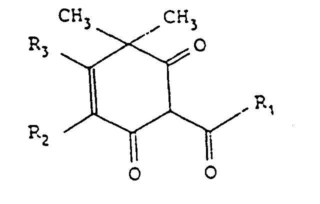 worin R₁ für einen C₁-C₆-Alkylrest, R₂ für ein Wasserstoffatom oder einen C₁-C₆-Alkylrest und R₃ für ein Wasserstoffatom, eine Hydroxygruppe oder einen C₁-C₆-Alkoxyrest steht.

7. Creme, Gel, Milch, Lotion oder Lippenstift nach Anspruch 6, dadurch gekennzeichnet, daß R₃ für einen C₁-C₆-Alkoxyrest steht.

8. Creme, Gel, Milch, Lotion oder Lippenstift nach Anspruch 6 oder 7, dadurch gekennzeichnet, daß R₃ für einen Methoxyrest steht.

9. Creme, Gel, Milch, Lotion oder Lippenstift nach Anspruch 6, 7 oder 8, dadurch gekennzeichnet, daß R₁ für einen Methyl- oder Isopropylrest, R₂ für ein Wasserstoffatom oder einen Methylrest und R₃ für einen Methoxyrest steht.

10. Creme, Gel, Milch, Lotion oder Lippenstift nach Anspruch 6, dadurch gekennzeichnet, daß die Verbindung der Formel I ausgewählt ist aus:
- 4,6,6-Trimethyl-5-methoxy-2-acetylcyclohex-4-en-1,3-dion,
- 4,6,6-Trimethyl-5-hydroxy-2-n-butyrylcyclohex-4-en-1,3-dion,
- 4,6,6-Trimethyl-5-methoxy-2-isobutyrylcyclohex-4-en-1,3-dion,
- 6,6-Dimethyl-5-methoxy-2-isobutyrylcyclohex-4-en-1,3-dion und
- 6,6-Dimethyl-2-isobutyrylcyclohex-4-en-1,3-dion.

11. 4,6,6-Trmethyl-5-methoxy-2-acetylcyclohex-4-en-1,3-dion der Formel (II): 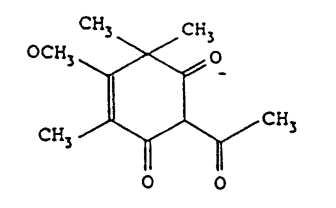 in einer beliebigen seiner verschiedenen tautomeren Formen.

12. Verfahren zur Herstellung von 4,6,6-Trimethyl-5-methoxy-2-acetylcyclohex-4-en-1,3-dion, dadurch gekennzeichnet, daß man
(a) den pH einer Lösung von alkalischen Enolaten des 4,6,6-Trimethyl-5-methoxy-2-acetylcyclohex-4-en-1,3-dion so einstellt, daß die entsprechenden Enole freigesetzt werden;
(b) die Enole durch Extraktion mittels eines Lösungsmittels isoliert und
(c) gegebenenfalls die Enole schließlich durch fraktionierende Destillation unter vermindertem Druck reinigt.

13. Verfahren nach Anspruch 12, dadurch gekennzeichnet, daß die Lösung der alkalischen Enolate nach einem Verfahren hergestellt wird, das aus den folgenden Schritten besteht:
(d) man unterzieht Blätter von Melaleuca cajeputi, entweder in frischer oder trockener Form, einer Wasserdampfdestillation, oder in teilweise getrockneter Form einer Extraktion mit einem Lösungsmittel;
(e) man unterzieht das in Schritt (d) erhaltene Produkt in Lösung in einem Lösungsmittel einer Extraktion mit einem Alkalicarbonat.
